(19) Europäisches Patentamt
European Patent Office
Office européen des brevets

(11) **EP 1 227 425 B1**

(12) **EUROPÄISCHE PATENTSCHRIFT**

(45) Veröffentlichungstag und Bekanntmachung des Hinweises auf die Patenterteilung:
**31.08.2005 Patentblatt 2005/35**

(51) Int Cl.7: **G06F 19/00**, G06F 17/00, A61B 5/04

(21) Anmeldenummer: **02001346.2**

(22) Anmeldetag: **18.01.2002**

(54) **Vorrichtung zum Auffinden der Auslöser anfallsartig auftretender Krankheiten**

Device for finding the triggers of paroxysmally occurring illnesses

Système pour découvrir la source des attaques de maladies

(84) Benannte Vertragsstaaten:
**DE FR GB IT NL**

(30) Priorität: **30.01.2001 DE 10103947**

(43) Veröffentlichungstag der Anmeldung:
**31.07.2002 Patentblatt 2002/31**

(73) Patentinhaber: SIEMENS AKTIENGESELLSCHAFT
80333 München (DE)

(72) Erfinder:
• **Abraham-Fuchs, Klaus**
91058 Erlangen (DE)
• **Rumpel, Eva, Dr.**
91052 Erlangen (DE)
• **Schmidt, Kai-Uwe, Dr.**
81541 München (DE)

(56) Entgegenhaltungen:
WO-A-99/63886          US-A- 3 841 309
US-A- 4 844 086        US-A- 5 857 980
US-A- 6 101 409

• CZINEGE L ET AL: "Multi-channel EEG activity correlation analysis to detect the onset of cerebral ischemia" ENGINEERING IN MEDICINE AND BIOLOGY SOCIETY, 1994. ENGINEERING ADVANCES: NEW OPPORTUNITIES FOR BIOMEDICAL ENGINEERS., PROCEEDINGS OF THE 16TH ANNUAL INTERNATIONAL CONFERENCE OF THE IEEE BALTIMORE, MD, USA 3-6 NOV. 1994, NEW YORK, NY, USA,IEEE, US, 3. November 1994 (1994-11-03), Seiten 1230-1231, XP010145642 ISBN: 0-7803-2050-6

**EP 1 227 425 B1**

## Beschreibung

Die Erfindung bezieht sich auf eine Vorrichtung zum Auffinden unbekannter, multifaktorieller Auslöser anfallsartig auftretender Krankheiten, die mit einer elektronischen Patientenakte und - gegebenenfalls räumlich verteilten - Datenbanken, z. B. einem elektronischen Patiententagebuch, einer Wetterdatenbank oder einem Expertensystem, in dem Regeln implementiert sind, die dem medizinischen Kenntnisstand über die anfallsartig auftretenden Krankheiten und deren Auslöser entsprechen, gekoppelt ist, wobei bereits Daten für die Zeiträume vor mehreren Anfällen vorliegen

Verschiedene Krankheiten wie Epilepsie oder Neurodermitis oder auch allergische Reaktionen zeichnen sich dadurch aus, dass sie aufgrund ihrer äußeren Ausprägung zweifelsfrei diagnostiziert werden können, es aber äußerst problematisch bleibt, den genauen Auslöser für die immer wiederkehrenden Schübe zu identifizieren. Die Suche nach dem kausalen Zusammenhang wird besonders erschwert, wenn nicht eine Substanz beziehungsweise Situation, sondern das Zusammentreffen von mehreren Faktoren das auslösende Moment darstellt. Bei - vorsichtig geschätzten - 20.000 allergenen Substanzen ergibt sich so eine unüberschaubare Menge an Möglichkeiten, die auszutesten weder durchführbar noch einem Patienten zumutbar ist. Der Erfolg einer einfachen Beobachtung dagegen ist zudem dadurch eingeschränkt, dass typischerweise zwischen einzelnen Anfällen Monate und Jahre vergehen.

Aus der WO 99/63886 ist bereits ein Informationsmanagementsystem vorbekannt, bei dem aus am Patienten erhobenen Datensätzen zu einem aktuellen Zeitpunkt durch Vergleich mit Normdatensätzen oder aber Datensätzen eines früheren Untersuchungszeitpunktes Änderungen erkannt werden können, um daraus eine Diagnose über die Krankheit abzuleiten. Dies hat aber mit einem System zur Erkennung der Auslöser einer anfallartigen Krankheit nichts zu tun, da jeweils die Daten beispielsweise beim Vergleich dieser Daten überhaupt kein zeitlicher Zusammenhang bezüglich der Erhebung der Daten zum Zeitpunkt eines Anfalls vorhanden ist. Bei dieser Entgegenhaltung werden bestimmte Datenwerte, also Blutdruck, Herzfunktion oder dergleichen, zu verschiedenen Zeitpunkten miteinander verglichen. Es wird aber nicht festgestellt, wie solche Daten im zeitlichen Zusammenhang mit einem nahe danach erfolgenden Anfall stehen. Demzufolge kann die Datenauswertung nach dieser WO 99/63886 auch keinerlei Aufschlüsse über die Ursache eines Anfalls liefern, sollten diese Erhebungen tatsächlich bei einem Anfallpatienten gemacht werden.

Der Erfindung liegt daher die Aufgabe zugrunde, eine Vorrichtung zum Auffinden unbekannter multifaktorieller Auslöser anfallsartig auftretender Krankheiten zu schaffen, die ohne aufwändige Immuntests auskommt.

Zur Lösung dieser Aufgabe ist erfindungsgemäß eine Vorrichtung der eingangs genannten Art gekennzeichnet durch eine Vergleichs-Auswerteeinrichtung, die nach einem Anfall eine Korrelationsanalyse zum Aufsuchen typischer Muster im zeitlichen Auftreten der gespeicherten Datenelemente in den verschiedenen Datenbanken im Zeitraum vor dem aktuellen Anfall und den Zeiträumen vor allen vorhergehenden Anfällen durchführt, wobei die Korrelationsanalyse, beispielsweise eine Hauptkomponentenanalyse, einen Clusteralgorithmus oder aber auch eine Auswertung mit Hilfe eines Assoziativspeichers umfassen kann.

Aus der WO 97/20496 A1 ist bereits eine Vorrichtung bekannt geworden, mithilfe deren eine automatische medizinische Diagnose erstellt sowie gegebenenfalls kontraindizierte Behandlungsstrategien erarbeitet werden sollen, wobei in einer ersten Datenbank Korrelationen einer Vielzahl von Krankheiten mit einer Vielzahl von Indikatoren, die mit jeder dieser Krankheiten verbunden sind, abgespeichert sind, während eine zweite Datenbank menschliche experimentelle Testergebnisse zu jedem Indikator enthält, wobei dann die Testergebnisse eines Patienten mit der zweiten Datenbank verglichen werden, um das Vorhandenseinniveau jedes Indikators zu bestimmen. Anschließend werden diese Anwesenheitsniveaus mit den Daten der ersten Datenbank verglichen, um ein Muster zu erhalten, aus dem sich das Vorhandensein der einen oder anderen Krankheit ergibt.

Dabei besteht aber ein grundsätzlicher Unterschied zur erfindungsgemäßen Vergleichs- und Auswertevorrichtung, da schon die Worte Indikator bei der vorliegenden Anmeldung etwas anderes bezeichnen, als bei der genannten WO 97/20496 A1. Die Indikatoren bei dieser Druckschrift beziehen sich auf bestimmte Krankheitssymptome, die mit einer Krankheit verbunden sind, also Ausprägungsformen der Krankheit selbst, während unter Indikatoren im Sinne der vorliegenden Anmeldung auslösende Faktoren verstanden werden, die meist in Verbindung mit weiteren Faktoren, zum Auslösen eines anfallartigen Ereignisses, also beispielsweise eines epileptischen Anfalls oder eines Migräneanfalls, führen. Bei der WO 97/20496 A1 werden die als Indikatoren bezeichneten Krankheitssymptome beim Patienten in einer automatisierten Form ausgewertet, um aus den Symptomen auf eine bestimmte Krankheit zu schließen, also um eine Krankheitsdiagnose zu stellen. Bei der vorliegenden Anmeldung geht es darum, eine vollständig klare Krankheit, nämlich eine bestimmte anfallartige Erkrankung wie Epilepsie, Migräneanfälle od. dgl., aufgrund der jeweiligen Vorgeschichte im Tagesablauf des Anfallpatienten dahingehend zu analysieren, welche Ursachen bei diesem Patienten jeweils den Anfall auslösen. Dies sind aber völlig verschiedene Ansatzpunkte einerseits und auf der anderen Seite werden auch völlig andere Lösungsmittel eingesetzt.

Alle zur Verfügung stehenden Daten aus verschiedenen, häufig auch räumlich verteilten, Datenbanken wie einer elektronischen Patientenkarte und besonders vorteilhaft auch einem elektronischen Patiententagebuch, werden zusammengeführt und auf ihren zeitlichen Zusammenhang mit den dokumentierten Anfällen hin untersucht. Die erfindungsgemäß durchgeführte Korrelationsanalyse vermag typische Muster im zeitlichen Auftreten der Datenelemente zu erkennen und damit anfallsauslösende Substanzen oder Situationen zu identifizieren.

In diesem Zusammenhang hat es sich auch als besonders zweckmäßig erwiesen, die erfindungsgemäße Vorrichtung an eine Wetterdatenbank anzuschließen, da klimatische Phänomene sehr häufig Ursache oder Mitursache von anfallsartigen Krankheitsschüben sind.

Neben dem für das Funktionieren der erfindungsgemäßen automatisierten Auffindung der Auslöser der anfallsartig auftretenden Krankheiten sehr wichtigen Patiententagebuch, in welchem Selbstbeobachtungen des Patienten niedergelegt sind, also beispielsweise was er jeweils macht, ob er sich im Freien oder im Haus aufhält und so weiter, was beispielsweise im Hinblick auf die Identifizierung von Pollenflug als Anfallsursache von Bedeutung sein kann, können auch Eintragungen des Personals des Krankenhauses, etwaige Pflegedienste oder dergleichen in einer elektronischen Patientenakte wichtige Hinweise auf die Krankheitsauslöser geben.

Das Wesen der vorliegenden Erfindung liegt dabei gegenüber der bisherigen einfachen Auswertung der elektronischen Patientenakten und Patiententagebüchern in der gegebenenfalls ohne Vorwissen erfolgenden Suche nach Mustern in Zeitreihen der Datenelemente, um aufgrund dieser Muster der Datenelemente für die jeweiligen Anfallsschübe Korrelationen und damit Hinweise auf auslösenden Ursachen zu erhalten.

Der Vorteil der erfindungsgemäßen Vorrichtung liegt darin, dass sie

a) die Analyse der Krankengeschichte über einen beliebig langen Zeitraum hinweg berücksichtigt,

b) auch Daten, die zu anderen Zwecken erhoben wurden, einbezogen werden können,

c) beliebig viele potentiell anfallsauslösende Faktoren in die Analyse einbezogen werden und

d) beliebig komplexe multifaktorielle Ursachen erkannt werden können.

Das System wird mit jedem neuen Eintrag in eine der Datenbanken aktiviert und ermöglicht so eine automatisierte und fortlaufende Suche nach den anfallsauslösenden Faktoren.

Erfindungsgemäß ist vorgesehen, dass die erfindungsgemäße Vorrichtung an ein Expertensystem angeschlossen ist, in dem Regeln implementiert sind, die den medizinischen Kenntnisstand über die anfallsartig auftretenden Krankheiten und deren Auslöser entsprechen, sodass nicht ausschließlich die vorstehend angesprochene, ohne Vorwissen erfolgende Suche nach Mustern in Zeitreihen der Datenelemente stattfindet, sondern zusätzlich zu diesen Mustern hinaus auch das bekannte Fachwissen über anfallsverursachende Auslösefaktoren mit berücksichtigt wird.

Ein solches System mit implementiertem Expertensystem kann - um einen entstehenden Verdacht weiter zu erhärten oder wieder zu verwerfen - auch weitere Informationen vom Patienten abfragen.

Auch bei dieser Ausführungsform mit einem Expertensystem wird dieses automatisch bei jedem neuen Eintrag in das Patiententagebuch aktiviert. Mithilfe der Expertensystems gewinnt jede einzelne neu hinzugekommene Information das ihren Verknüpfungen entsprechende Gewicht und sorgt somit für einen überproportionalen Erkenntnisgewinn. Der Einsatz von Expertensystemen zusätzlich zu der Vergleichsauswerteeinrichtung zum Suchen nach Mustern in den Zeitreihen der Datenelemente verspricht daher ein schnelleres und erfolgreicheres Auffinden anfallsauslösender Faktoren. Ein solches Expertensystem kann dabei zum Beispiel in Form eines Bayesschen Netzes oder eines neuronalen Netzes ausgestaltet sein.

Weitere Vorteile, Merkmale und Einzelheiten der Erfindung ergeben sich aus der nachfolgenden Beschreibung einiger Ausführungsbeispiele sowie anhand der Zeichnung. Dabei zeigen:

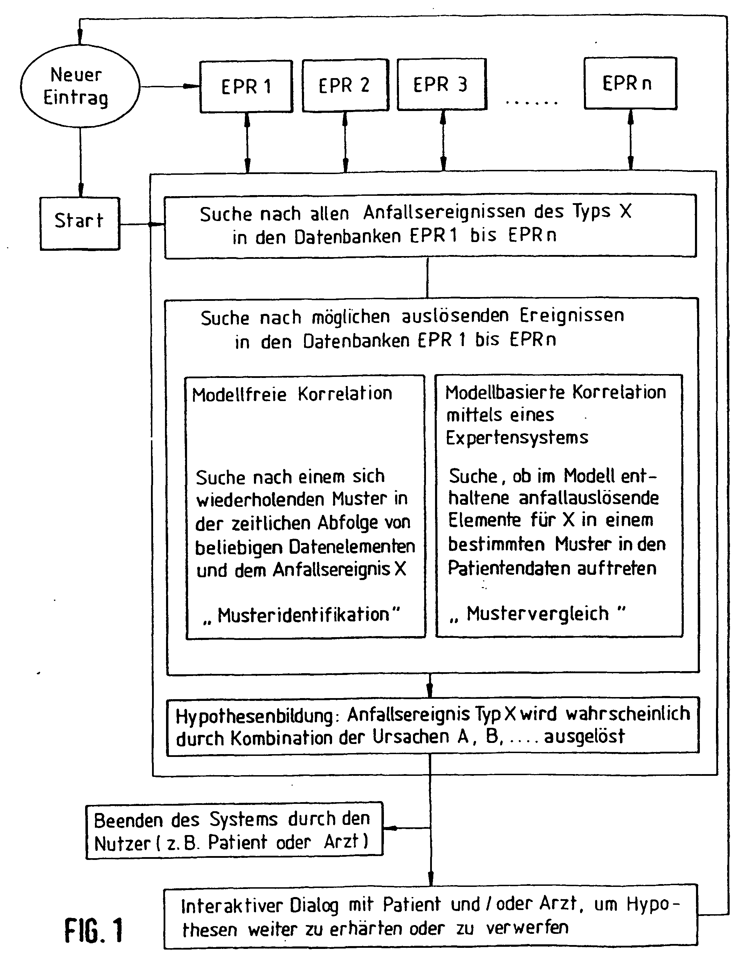

Fig. 1    ein Funktionsablaufdiagramm einer erfindungsgemäßen Vorrichtung ohne Expertensystem links und mit Expertensystem rechts,

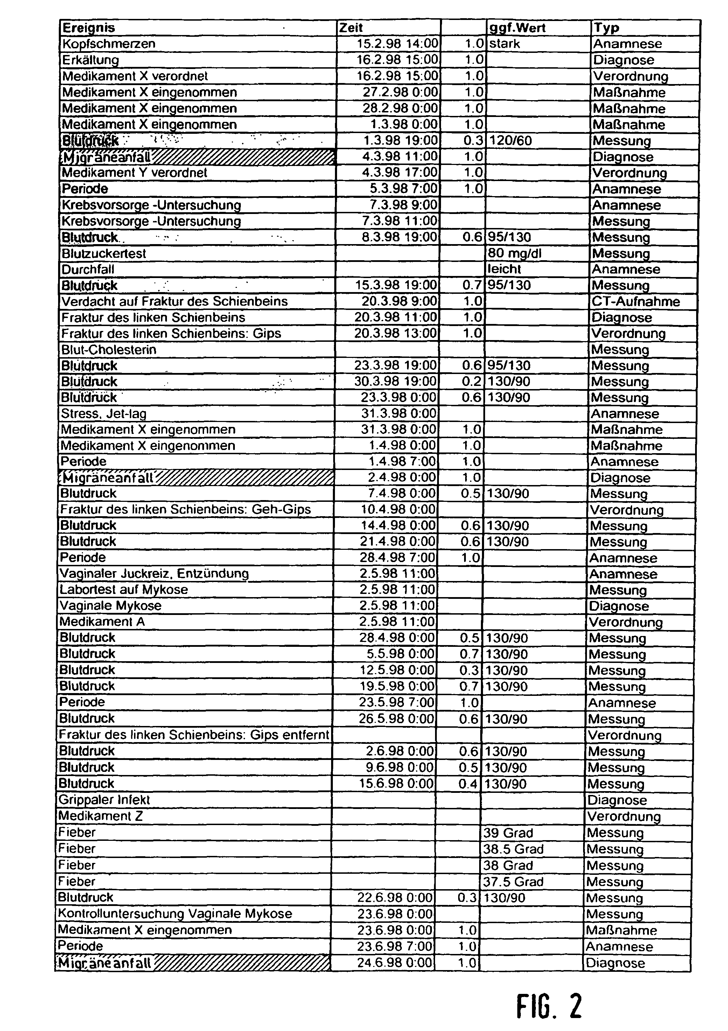

Fig. 2    ein Tagebuchauszug eines Migränepatienten über einen Zeitraum, in dem drei Migräneanfälle aufgetreten sind und

Fig. 3    eine Auswertediagramm, aus dem sich ergibt, wie durch eine bestimmte Indikatorengruppe der jeweilige Anfall ausgelöst wurde.

Zahlreiche mathematische Ansätze für das Auffinden von Mustern in mehrdimensionalen Datensätzen sind bekannt und auf die Problemstellung dieser Erfindung anwendbar. Hier sei nur zur Illustration eine mögliche Mustererkennungsstrategie genannt, die zum Einsatz kommen könnte.

Ereigniseinträge in eine EPR können im mathematischen Sinne als Matrizen oder Vektoren beschrieben werden, wobei unterschiedliche Werte-Dimensionen betrachtet werden können (Art des Ereignisses, Messwert des Ereignisses, Zeitpunkt des Auftretens). Als "Messwert" kann auch eingesetzt werden: 1 für ein Auftreten des Ereignisses, 0 für das Ausbleiben des Ereignisses. Eine Möglichkeit des vektoriellen Beschreibung ist, zu einem Zeitfenster (z. B. innerhalb eines Tages) die Wert einer vorgegebenen Ereignismengen als Ereignis-Vektor E(ti) zum Zeitpunkt ti darzustellen, z. B. für einen Ereignisvektor mit M Ereignissen (Dimension j=1...M).

$$
\overline{E}\ (ti) = \begin{matrix} \text{Blutdruck} \\ \text{Einnahme Medikament X} \\ \text{Einnahme Medikament Y} \\ \text{Periode} \\ \text{Migräneanfall} \\ \text{Stress} \\ \text{Fieber} \end{matrix} = \begin{matrix} 0,2 \\ 1 \\ 0 \\ 1 \\ 0 \\ 1 \\ 37 \end{matrix} = \begin{matrix} E1 \\ \cdot \\ \cdot \\ \cdot \\ \cdot \\ \cdot \\ EM \end{matrix}
$$

Die Ähnlichkeit zweier Ereignismuster E1 und E2 ist dann mathematisch beschreibbar als Winkel zwischen den Vektoren, was gleichbedeutend mit folgender methematischer Definition der Korrelation zwischen den Vektoren ist:

$$ C = \frac{E1 * E2}{E1 * E2} $$

Treten die gesuchten Auslöser z. B. als Kombination von Ereignissen in mehreren aufeinander folgenden Zeitscheiben (d. h. an mehreren aufeinanderfolgenden Tagen vor dem Anfall, siehe Beispiel) auf, so müssen mehrere Vektoren im interessierenden Zeitfenster Tn = T1...TN (z. B. 1....5 Tage) zu einem Set ("Template") zusammengefasst werden. Dieses Set wird dann als gleitender Vektor über den Evaluierungszeitraum geschoben und die gleitende Korrelationsfunktion berechnet. Sind die Ereignisse im Template und im Untersuchungsraum zufällig verteilt, so ergibt dies eine stark verrauschte Korrelationsfunktion über der Zeit mit niedrigen Korrelationswerten. Tritt jedoch ein Ereignismuster innerhalb des Templates im Evaluierungs-Zeitraum wiederholt auf, so ergibt dies einen Peak hoher Korrelation in der Korrelationsfunktion über der Zeit, wenn das Template über das gleiche oder ähnliche Ereignis geschoben wird. Anhand dieser Peaks kann das Auftreten ähnlicher Ereignisse unter Verwendung von Schwellwerten für die Korrelation automatisch bestimmt werden.

Die Berechnung der Korrelationsfunktion über der Zeit lässt sich mit diesem Modell mathematisch folgendermaßen beschreiben:

$$ C(ti) = \frac{\sum_{j=1}^{M} \sum_{Tn=1}^{N} E_j(ti+Tn) * E_j(Tn)}{\sum_{j=1}^{M} \sum_{Tn=1}^{N} E_j^2(ti+Tn) * \sum_{j=1}^{M} \sum_{Tn=1}^{N} E_j^2(Tn)} $$

Unterschiedliche Strategien zur automatischen Suche von Ereignismustern, die Auslöser des Anfalls sind, können angewandt werden. Man kann z. B. einen Anfall herausgreifen, ein Zeitfenster von 3 Tagen vor dem Anfall als Template definieren, nach dem Auftreten von Korrelationspeaks vor weiteren Anfällen suchen, und dann kombinatorisch den Ereignisraum (=Dimension des Vektors) verkleinern, um die Ereignisse herauszufiltern, die den Korrelationspeak verursacht haben. Mit den Datenmengen in den hier betrachteten Dimensionen und Zeiträumen sind Korrelationsfunktionen über lange Zeiträume (Monate und Jahre) in wenigen Sekunden bis Sekundenbruchteilen berechenbar, sodass diese Suchstrategie realistisch automatisiert durchführbar ist.

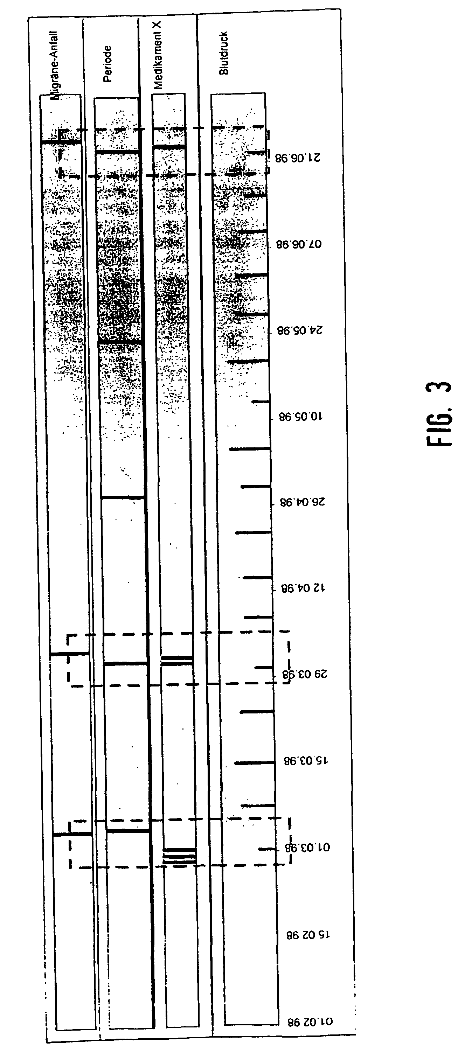

In dem beigefügten Beispiel ist in Fig. 3 erkennbar, dass die kombinierten Auslöser für einen Migräneanfall des Patienten die Einnahme des Medikamentes X und niedriger Blutdruck kurz vor Eintreten der weiblichen Periode ist. Treten diese Ereignisse nicht gemeinsam auf, kommt es auch nicht zum Migräneanfall.

**Patentansprüche**

1. Vorrichtung zum Auffinden unbekannter, multifaktorieller Auslöser anfallsartig auftretender Krankheiten, die mit einer elektronischen Patientenakte und - gegebenenfalls räumlich verteilten - Datenbanken, z. B. einem elektronischen Patiententagebuch, einer Wetterdatenbank oder einem Expertensystem, in dem Regeln implementiert sind, die dem medizinischen Kenntnisstand über die anfallsartig auftretenden Krankheiten und deren Auslöser entsprechen, gekoppelt ist, wobei bereits Daten für die Zeiträume vor mehreren Anfällen vorliegen, **gekennzeichnet durch** eine Vergleichs-Auswerteeinrichtung, die nach einem Anfall eine Korrelationsanalyse zum Aufsuchen typischer Muster im zeitlichen Auftreten der gespeicherten Datenelemente in den verschiedenen Datenbanken im Zeitraum vor dem aktuellen Anfall und den Zeiträumen vor allen vorhergehenden Anfällen durchführt.

2. Vorrichtung nach Anspruch 1, **dadurch gekennzeichnet, dass** die Korrelationsanalyse eine Hauptkomponentenanalyse ist.

3. Vorrichtung nach Anspruch 1, **dadurch gekennzeichnet, dass** die Korrelationsanalyse einen Clusteralgorithmus beinhaltet.

4. Vorrichtung nach Anspruch 1, **dadurch gekennzeichnet, dass** die Vergleichs-Auswerteeinrichtung einen Assoziativspeicher enthält.

5. Vorrichtung nach einem der Ansprüche 1 bis 4, **dadurch gekennzeichnet, dass** das Expertensystem ein Bayessches Netzwerk enthält.

6. Vorrichtung nach einem der Ansprüche 1 bis 4, **dadurch gekennzeichnet, dass** das Expertensystem ein neuronales Netz enthält.

**Claims**

1. Device for finding unknown, multifactorial triggers of paroxysmally occurring illnesses, which is coupled to an electronic patient file and - if appropriate, spatially distributed - databases, e.g. an electronic patient diary, a weather database or an expert system, in which rules are implemented which correspond to the state of medical knowledge on the paroxysmally occurring illnesses and their triggers, data for the periods of time before a plurality of paroxysms already being present, **characterized by** a comparison and evaluation device which carries out, after a paroxysm, a correlation analysis in order to search for typical patterns in the temporal occurrence of the stored data elements in the various databases in the period of time before the present paroxysm and the periods of time before all the preceding paroxysms.

2. Device according to Claim 1, **characterized in that** the correlation analysis is a main component analysis.

3. Device according to Claim 1, **characterized in that** the correlation analysis includes a cluster algorithm.

4. Device according to Claim 1, **characterized in that** the comparison and evaluation device contains an associative memory.

5. Device according to one of Claims 1 to 4, **characterized in that** the expert system includes a Bayes network.

6. Device according to one of Claims 1 to 4, **characterized in that** the expert system includes a neural network.

**Revendications**

1. Dispositif pour découvrir des déclencheurs inconnus et multifactoriels de maladies qui se manifestent par des attaques, ce dispositif étant couplé à un dossier électronique de patient et à des bases de données (éventuellement disséminées dans l'espace), par exemple un journal électronique du patient, une base de données météorologique ou un système expert dans lequel sont mises en oeuvre des règles qui correspondent à l'état des connaissances médicales sur les maladies qui se manifestent par des attaques et sur leurs déclencheurs, des données étant déjà disponibles pour les périodes qui précèdent les attaques,

**caractérisé par** une installation d'exploitation et de comparaison qui procède après une attaque à une analyse de corrélation pour rechercher des modèles typiques dans la manifestation chronologique des éléments enregistrés dans les différentes bases de données sur une période qui précède l'attaque actuelle et sur les périodes qui précèdent toutes les attaques précédentes.

2. Dispositif selon la revendication 1, **caractérisé en ce que** l'analyse de corrélation est une analyse des composantes principales.

3. Dispositif selon la revendication 1, **caractérisé en ce que** l'analyse de corrélation est un algorithme de groupe.

4. Dispositif selon la revendication 1, **caractérisé en ce que** l'installation d'exploitation et de comparaison contient une mémoire associative.

5. Dispositif selon l'une des revendications 1 à 4, **caractérisé en ce que** le système expert contient un réseau de Bayes.

6. Dispositif selon l'une des revendications 1 à 4, **caractérisé en ce que** le système expert contient un réseau neuronal.

**FIG. 1**

Neuer Eintrag

EPR 1  EPR 2  EPR 3  ......  EPR n

Start

Suche nach allen Anfallsereignissen des Typs X in den Datenbanken EPR 1 bis EPR n

Suche nach möglichen auslösenden Ereignissen in den Datenbanken EPR 1 bis EPR n

Modellfreie Korrelation

Suche nach einem sich wiederholenden Muster in der zeitlichen Abfolge von beliebigen Datenelementen und dem Anfallsereignis X

„ Musteridentifikation"

Modellbasierte Korrelation mittels eines Expertensystems

Suche, ob im Modell enthaltene anfallauslösende Elemente für X in einem bestimmten Muster in den Patientendaten auftreten

„ Mustervergleich "

Hypothesenbildung: Anfallsereignis Typ X wird wahrscheinlich durch Kombination der Ursachen A, B, .... ausgelöst

Beenden des Systems durch den Nutzer ( z. B. Patient oder Arzt )

Interaktiver Dialog mit Patient und / oder Arzt, um Hypothesen weiter zu erhärten oder zu verwerfen

| Ereignis | Zeit | | ggf.Wert | Typ |
|---|---|---|---|---|
| Kopfschmerzen | 15.2.98 14:00 | 1.0 | stark | Anamnese |
| Erkältung | 16.2.98 15:00 | 1.0 | | Diagnose |
| Medikament X verordnet | 16.2.98 15:00 | 1.0 | | Verordnung |
| Medikament X eingenommen | 27.2.98 0:00 | 1.0 | | Maßnahme |
| Medikament X eingenommen | 28.2.98 0:00 | 1.0 | | Maßnahme |
| Medikament X eingenommen | 1.3.98 0:00 | 1.0 | | Maßnahme |
| Blutdruck | 1.3.98 19:00 | 0.3 | 120/60 | Messung |
| Migräneanfall | 4.3.98 11:00 | 1.0 | | Diagnose |
| Medikament Y verordnet | 4.3.98 17:00 | 1.0 | | Verordnung |
| Periode | 5.3.98 7:00 | 1.0 | | Anamnese |
| Krebsvorsorge -Untersuchung | 7.3.98 9:00 | | | Anamnese |
| Krebsvorsorge -Untersuchung | 7.3.98 11:00 | | | Messung |
| Blutdruck | 8.3.98 19:00 | 0.6 | 95/130 | Messung |
| Blutzuckertest | | | 80 mg/dl | Messung |
| Durchfall | | | leicht | Anamnese |
| Blutdruck | 15.3.98 19:00 | 0.7 | 95/130 | Messung |
| Verdacht auf Fraktur des Schienbeins | 20.3.98 9:00 | 1.0 | | CT-Aufnahme |
| Fraktur des linken Schienbeins | 20.3.98 11:00 | 1.0 | | Diagnose |
| Fraktur des linken Schienbeins: Gips | 20.3.98 13:00 | 1.0 | | Verordnung |
| Blut-Cholesterin | | | | Messung |
| Blutdruck | 23.3.98 19:00 | 0.6 | 95/130 | Messung |
| Blutdruck | 30.3.98 19:00 | 0.2 | 130/90 | Messung |
| Blutdruck | 23.3.98 0:00 | 0.6 | 130/90 | Messung |
| Stress, Jet-lag | 31.3.98 0:00 | | | Anamnese |
| Medikament X eingenommen | 31.3.98 0:00 | 1.0 | | Maßnahme |
| Medikament X eingenommen | 1.4.98 0:00 | 1.0 | | Maßnahme |
| Periode | 1.4.98 7:00 | 1.0 | | Anamnese |
| Migräneanfall | 2.4.98 0:00 | 1.0 | | Diagnose |
| Blutdruck | 7.4.98 0:00 | 0.5 | 130/90 | Messung |
| Fraktur des linken Schienbeins: Geh-Gips | 10.4.98 0:00 | | | Verordnung |
| Blutdruck | 14.4.98 0:00 | 0.6 | 130/90 | Messung |
| Blutdruck | 21.4.98 0:00 | 0.6 | 130/90 | Messung |
| Periode | 28.4.98 7:00 | 1.0 | | Anamnese |
| Vaginaler Juckreiz, Entzündung | 2.5.98 11:00 | | | Anamnese |
| Labortest auf Mykose | 2.5.98 11:00 | | | Messung |
| Vaginale Mykose | 2.5.98 11:00 | | | Diagnose |
| Medikament A | 2.5.98 11:00 | | | Verordnung |
| Blutdruck | 28.4.98 0:00 | 0.5 | 130/90 | Messung |
| Blutdruck | 5.5.98 0:00 | 0.7 | 130/90 | Messung |
| Blutdruck | 12.5.98 0:00 | 0.3 | 130/90 | Messung |
| Blutdruck | 19.5.98 0:00 | 0.7 | 130/90 | Messung |
| Periode | 23.5.98 7:00 | 1.0 | | Anamnese |
| Blutdruck | 26.5.98 0:00 | 0.6 | 130/90 | Messung |
| Fraktur des linken Schienbeins: Gips entfernt | | | | Verordnung |
| Blutdruck | 2.6.98 0:00 | 0.6 | 130/90 | Messung |
| Blutdruck | 9.6.98 0:00 | 0.5 | 130/90 | Messung |
| Blutdruck | 15.6.98 0:00 | 0.4 | 130/90 | Messung |
| Grippaler Infekt | | | | Diagnose |
| Medikament Z | | | | Verordnung |
| Fieber | | | 39 Grad | Messung |
| Fieber | | | 38.5 Grad | Messung |
| Fieber | | | 38 Grad | Messung |
| Fieber | | | 37.5 Grad | Messung |
| Blutdruck | 22.6.98 0:00 | 0.3 | 130/90 | Messung |
| Kontrolluntersuchung Vaginale Mykose | 23.6.98 0:00 | | | Messung |
| Medikament X eingenommen | 23.6.98 0:00 | 1.0 | | Maßnahme |
| Periode | 23.6.98 7:00 | 1.0 | | Anamnese |
| Migräneanfall | 24.6.98 0:00 | 1.0 | | Diagnose |

## FIG. 2

FIG. 3